# EUROPEAN PATENT APPLICATION

(11) **EP 3 706 137 A1**
(43) Date of publication of application: **09.09.2020**
(21) Application number: 19161587.1
(22) Date of filing: 08.03.2019
(51) Int. Cl.: G16H 30/40, G16H 50/50

(54) **METHOD AND SYSTEM FOR PATIENT-SPECIFIC PREDICTING OF CYCLIC LOADING FAILURE OF A CARDIAC IMPLANT**

(71) Applicant: FEops NV, 9052 Gent (BE)
(72) Inventor: de Bock, Sander, 9052 Gent (BE); De Santis, Gianluca, 9052 Gent (BE); Iannaccone, Francesco, 9052 Gent (BE)
(74) Representative: V.O.

(57) **Abstract**

A method and system for patient-specific predicting of cyclic loading failure of a cardiac implant. The method includes providing an implant model representing a three dimensional mesh based representation of a cardiac implant, and providing a four-dimensional, 4D, patient-specific anatomical model representing a mesh based representation of a patient-specific cardiac region including a deployment site for the cardiac implant in a plurality of states corresponding to a plurality of moments in the cardiac cycle. A computer calculates deformation of the implant model deployed at the deployment site when, or before, the 4D patient-specific anatomical model transforms consecutively through the plurality of states, and the computer determines a risk of cyclic loading failure of the cardiac implant on the basis of the calculated implant deformation.

## Description

### FIELD OF THE INVENTION

The present invention relates to the field of pre-operative planning of transcatheter structural heart interventions, e.g. valve treatment, such as valve implantation and/or repair. More in particular, the invention relates to pre-operative prediction of cyclic loading failure of a cardiac implant.

### BACKGROUND TO THE INVENTION

WO2013/171039A1 describes a method for preoperative insights into the interaction of an implant device and specific patient anatomy, for better prediction of complications, such as regurgitation, for better prediction of the hemodynamic performance of an implant deployed in an aortic valve, and for better patient selection and stratification.

WO2018/141927A1 describes a method for predicting a measure of hemodynamic compromise as an acute result of transcatheter structural heart intervention, e.g. at a plurality of moments during the cardiac cycle. Hemodynamic compromise after deployment can also determined.

Although these provides major advances in preoperative planning, and improved possibilities for selecting a suitable implant device for a specific patient to improve acute outcome, there still is a need for more information on the chronic interaction of an implant device and specific patient anatomy to further enhance preoperative planning. It would be of great value if the long term remodeling of the heart, being the heart anatomy changing due to the prolonged presence of the implant, could be taken into account when evaluating chronic outcome of the intervention.

### SUMMARY OF THE INVENTION

Implant devices in or at the heart, or other moving organs, are subjected to repeated deformation or repeated loading. For example, a cardiac implant can be subject to cyclic loading due to the cardiac cycle. During systole a cardiac implant can be compressed, whereas during diastole the cardiac implant may relax. It has been found that an implant device that has been determined as optimal in view of acute outcome (e.g. deployment) not necessarily is optimal in view of chronic outcome (e.g. cyclic loading failure, such as high cycle fatigue fracture). Moreover, it has been found that performance of an implant device in view of cyclic loading failure can be highly dependent on the patient-specific cyclic loading imposed by the patient-specific anatomy.

Therefore, according to the invention is provided a method for patient-specific predicting of cyclic loading failure of a cardiac implant. The method of predicting can be performed before actual deployment of a real cardiac implant device into the patient anatomy. The method includes providing an implant model representing a three-dimensional mesh based representation of a cardiac implant. The method also includes providing a four-dimensional, 4D, patient-specific anatomical model representing a mesh-based representation of a patient-specific cardiac region including a deployment site for the cardiac implant in a plurality of states corresponding to a plurality of moments in the cardiac cycle. According to the method, a computer calculates deformation of the implant model deployed at the deployment site when the 4D patient-specific anatomical model transforms consecutively through the plurality of states, e.g. using finite element analysis, and determines a risk of cyclic loading failure of the cardiac implant on the basis of the calculated implant deformation and deformation history. This provides the advantage that cyclic loading failure for the implant device can be predicted on the basis of the patient-specific anatomy and before deployment of the implant device.

Optionally, the method includes determining a risk of cyclic loading failure of the cardiac implant on the basis of the calculated implant deformation and data representative of deformation history. Thus, data representative of deformation history can be taken into account. Data representative of deformation history can e.g. include a time duration, number of cycles, predicted implant device migration, predicted anatomical changes over time, etc., and implant deformation during loading into catheter and advancing the implant towards the deployment site, time duration, number of cycles, predicted implant device migration, predicted anatomical changes over time, etc..

Optionally, the method includes after deployment of the implant model at the deployment site, calculating deformation of the implant model while the 4D patient-specific anatomical model transforms consecutively through the plurality of states, . The implant model may have been deployed at the deployment site without modeling beating of the heart during deployment. Alternatively, the implant model may have been deployed at the deployment site while modeling beating of the heart during deployment. The method can include calculating deformation of the implant model during implant model deployment, while the 4D patient-specific anatomical model transforms consecutively through the plurality of states.

Optionally, the 4D patient-specific anatomical model represents a mesh-based representation of a patient-specific cardiac region in a plurality of states corresponding to a plurality of moments in the cardiac cycle before deployment. Hence, nodes of the 4D anatomical model transforms through the plurality of states following displacement of the patient-specific cardiac region in the absence of an implant device.

The deformation of the patient-specific anatomical model representing the patient-specific cardiac region is imposed onto the implant device through contact between the anatomical model and the implant model. Hence, deformation of the patient-specific cardiac region as a result of the presence of the implant device is not taken into account. It has been found that this can provide a good approximation for relatively flexible implant devices, i.e. devices that are much more flexible as compared to the hosting anatomy.

Optionally, a 4D patient-specific intermediate model is provided, having its nodes associated with nodes of the 4D patient-specific anatomical model. The 4D patient-specific intermediate model represents a mesh-based representation of a patient-specific cardiac region in a plurality of states corresponding to a plurality of moments in the cardiac cycle before deployment. Hence, the associated nodes of the 4D intermediate model transform through the plurality of states following displacement of the patient-specific cardiac region in the absence of an implant device. Then, the 4D patient-specific anatomical model transforms through the plurality of states through imposing the displacements of associated nodes of the 4D intermediate model via stiffness or dashpot elements connecting the associated nodes of the 4D intermediate model to the nodes of the anatomical model. Hence, the deformation of the 4D patient-specific intermediate model is driven by the nodes, of the intermediate model, associated to the nodes of the anatomical model, while the nodes of anatomical model can (resiliently) move relative to the associated nodes of the intermediate model. Deformation of the implant model is achieved by contact between 4D patient-specific anatomical model and the implant model. Hence deformation of the patient-specific cardiac region as a result of the presence of the implant device is taken into account. It has been found that this can provide a good approximation for relatively stiff implant devices, i.e. devices that deform the hosting anatomy. It will be appreciated that it can be verified, e.g. through comparative testing, which of these two options (impose deformation directly on anatomical nodes or indirectly using associated nodes connected to anatomical nodes via mechanical stiffness and/or dashpot elements) best suits the implant device under investigation.

Optionally, the 4D patient-specific anatomical model has mechanical properties, including stiffness and/or viscosity. In such a case the overall mechanical behavior of the 4D patient-specific anatomical model depends on the combination of the mechanical properties of the 4D anatomical model, mechanical properties of the stiffness and/or dashpot elements connecting associated nodes to nodes of the 4D anatomical model.

Optionally, the method includes determining mechanical stress and/or strain within each mesh element of the implant model. Optionally, the method includes determining mechanical stress and/or strain within each mesh element of the implant model for each of the plurality of states in the course of the cardiac cycle. The mechanical stress and/or strain within the mesh elements of the implant model is representative for cyclic loading fatigue. The mechanical stress and/or strain within the mesh elements of the implant model, and the variation thereof during the entire cycle, is representative for cyclic loading fatigue.

Optionally, the method includes determining for each mesh element of the implant model an amplitude of the mechanical stress and/or strain occurring in the course of the cardiac cycle. The determined amplitudes are representative of the risk of cyclic loading failure.

Optionally, the cardiac implant is a valve implant, stent, or the like.

Optionally, the cyclic loading failure includes one or more of the following: high cycle fatigue fracture, such as material fatigue, implant migration, valve failure, or the like.

Optionally, if the cyclic loading simulation can predict a first fracture. A second (post-fracture) cyclic loading simulation can be performed using the fractured implant model and optionally modifying the anatomical model and/or the loading conditions. This can be repeated multiple times to evaluate the impact of subsequent cyclic loading failures.

Optionally, the risk of cyclic loading failure is determined by means of an S-N curve, as is known in the art. Optionally, the risk of cyclic loading failure is determined by means of a rainflow-counting algorithm, as is known in the art. Optionally, the risk of cyclic loading failure is determined by strain amplitude (e.g. for Nitinol components).

Optionally, the method includes providing the 4D patient-specific anatomical model, or its associated nodes, on the basis of one or more of a) segmentation of a 4D preoperative image, b) landmarks in 4D preoperative images, c) a 3D or 4D preoperative image in combination with patient-specific measurements of blood volume, flow and/or pressure taken before and/or after deployment of the implant, d) a 3D or 4D preoperative image in combination with non-patient specific knowledge of patho-physiology of the heart, such as e.g. expected motion, or e) a 3D or 4D preoperative image in combination with non-patient specific knowledge of heart remodeling at chronic stage, such as tissue overgrowth limiting motion of the implant occurring weeks or months after treatment.

According to an aspect, the method includes receiving a plurality of, at least quasi, three-dimensional, 3D, medical images representing the patient-specific cardiac region in the plurality of states corresponding to the plurality of moments in the cardiac cycle, and constructing the 4D patient-specific anatomical model on the basis thereof. Hence, the 4D patient-specific anatomical model can be constructed efficiently from available medical images.

Optionally, the method includes constructing a 3D mesh based representation of the patient-specific cardiac region on the basis of one of the medical images, determining a transformation from one medical image to the next, and applying the transformation to the constructed 3D mesh based representation, for providing the 4D patient-specific anatomical model. The 3D mesh based representation represents a 3D patient-specific anatomical model of the patient-specific cardiac region. Thus, only a single 3D mesh based representation needs be constructed, and the transforming of the 4D patient-specific anatomical model (or the 4D patient-specific intermediate model) consecutively through the plurality of states is obtained from the transforming of the medical images through the plurality of states (e.g. using elastic registration algorithms). It will be appreciated that the transforming of the medical images through the plurality of states can make use of a 4D vector field.

Optionally, the method includes for each of the medical images constructing a 3D mesh based representation of the patient-specific cardiac region, determining a transformation from one 3D mesh based representation to the next, and determining the 4D patient-specific anatomical model on the basis of the plurality of 3D mesh based representations (e.g. by first determining the 4D patient-specific intermediate model on the basis of the plurality of 3D mesh based representations). It will be appreciated that the transforming of the meshes through the plurality of states can make use of a 4D vectors only at the nodes of the mesh.

Optionally, the method includes deploying an implant model at a plurality of different positions in the 4D patient-specific anatomical model, determining a risk of cyclic loading failure for each of the positions, and selecting the position associated with the lowest risk for real-life implantation. Hence, an optimum implant device location can be determined in view of minimizing a risk of cyclic loading failure.

Optionally, the method includes providing a plurality of different implant models, each implant model representing geometrical (e.g. including size of the same implant device) and/or material properties of a corresponding real-life implant device, determining a risk of cyclic loading failure of the cardiac implant for each implant model, and selecting the implant device associated with the implant model for which the lowest risk was calculated, for real-life implantation. Hence, an optimum implant device can be chosen in view of minimizing a risk of cyclic loading failure. It will be appreciated that for each of the plurality of different implant models, a plurality of different deployment locations may be used.

According to an aspect is provided a method for designing bench loading conditions for experimental fatigue testing (accelerated wear testing, or fatigue machine). The highest risk of cyclic loading failure can be used to design the bench and calibrate the loading conditions for experimental fatigue testing. From patient-specific deformation or stress or strain of the implant device, the condition leading to highest risk of cyclic loading failure can be reproduced with a simplified bench that runs accelerated cycles and allows to test the implant device fatigue safety within a time period shorter than the in-vivo time period, by performing cycles at higher frequency.

According to an aspect is provided a system for patient-specific predicting of cyclic loading failure of a cardiac implant. The system includes a processor. The processor is arranged for receiving an implant model representing a three dimensional mesh based representation of a cardiac implant. The processor is arranged for receiving a four-dimensional, 4D, patient-specific anatomical model representing a mesh based representation of a patient-specific cardiac region including a deployment site for the cardiac implant in a plurality of states corresponding to a plurality of moments in the cardiac cycle. The processor is arranged for calculating deformation of the implant model deployed at the deployment site when or before that the 4D patient-specific anatomical model transforms consecutively through the plurality of states. The processor is arranged for determining a risk of cyclic loading failure of the cardiac implant on the basis of the calculated deformation.

According to an aspect is provided a computer program product including computer implementable instructions which when implemented by a programmable computer causes the computer to retrieve an implant model representing a three dimensional mesh based representation of a cardiac implant; retrieve a four-dimensional, 4D, patient-specific anatomical model representing a mesh based representation of a patient-specific cardiac region including a deployment site for the cardiac implant in a plurality of states corresponding to a plurality of moments in the cardiac cycle; calculate deformation of the implant model deployed at the deployment site when the 4D patient-specific anatomical model transforms consecutively through the plurality of states; and determine a risk of cyclic loading failure of the cardiac implant on the basis of the calculated deformation.

It will be appreciated that all features and options mentioned in view of the method apply equally to the system and the computer program product. It will also be clear that any one or more of the above aspects, features and options can be combined.

### BRIEF DESCRIPTION OF THE DRAWINGS

Embodiments of the present invention will now be described in detail with reference to the accompanying drawings in which:
Figure 1 is schematic representation of a system;
Figure 2 is a schematic representation of a method;
Figure 3 is a schematic representation of a method;
Figure 4 is a schematic representation of a method; and
Figures 5A-5D are exemplary views of models.

### DETAILED DESCRIPTION

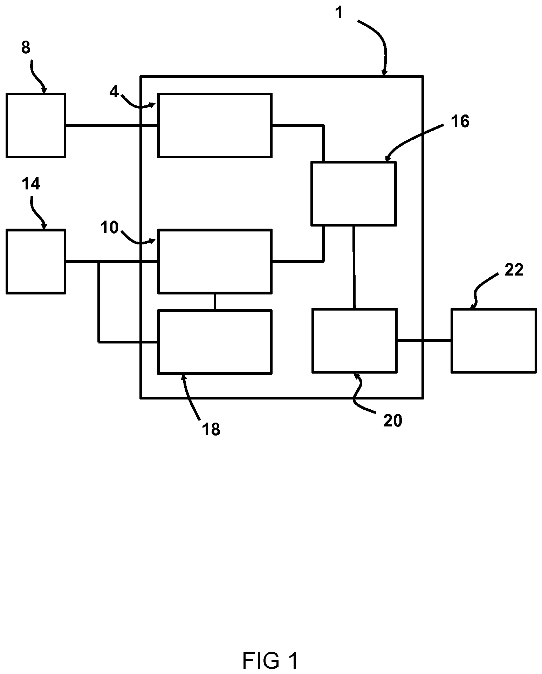

Figure 1 shows a schematic representation of an exemplary system 1 for patient-specific predicting of cyclic loading failure of an implant device 2. In this example the implant device 2 is a cardiac implant, such as a stent or a replacement valve.

The system 1 includes a first receiving unit 4 arranged for receiving an implant model 6. The first receiving unit 4 can receive the implant model 6 from a first source 8, such as a memory, a database, a network such as the internet, or the like. The implant model 6 represents a three dimensional, 3D, mesh based representation of an implant device 2. The system 1 includes a second receiving unit 10 arranged for receiving a four-dimensional, 4D, patient-specific anatomical model 12. The second receiving unit 10 can receive the 4D patient-specific anatomical model 12 from a second source 14, such as a memory, a database, a network such as the internet, a medical imaging device such as a CT device, MRI device, ultrasound device or the like. The 4D patient-specific anatomical model represents a mesh based representation of a patient-specific anatomical region including a deployment site for the implant device in a plurality of states corresponding to a plurality of moments in a movement cycle. The states represent consecutive moments during the movement cycle. Therefore, here the 4D patient-specific anatomical model can be seen as a 3D patient-specific anatomical model wherein the fourth dimension of time is added, represented by a plurality of discrete states. Thus the 3D patient-specific anatomical model deforms from one state to the next. In this example, the 4D patient-specific anatomical model 12 represents a mesh based representation of a patient-specific cardiac region including a deployment site for the cardiac implant in a plurality of states corresponding to a plurality of moments in the cardiac cycle. It will be appreciated that 4D patient-specific anatomical model 12 includes at least two states, e.g. a first state associated with systole and a second state associated with diastole. Preferably, the 4D patient-specific anatomical model 10 includes more states in the cycle, such as 3, 4, 5, 6, 8, 10, 12, 16, 20, 24, 32, 64 or 128 states. However, other numbers of states in the cycle are possible as well.

The system 1 includes a processor 16. The processor is arranged for virtually deploying the implant model 6 at the deployment site. The processor 16 is arranged for calculating deformation of the deployed implant model 6 while the 4D patient-specific anatomical model 12 transforms consecutively through the plurality of states. The processor 16 is also arranged for determining a risk of cyclic loading failure of the implant device 2 on the basis of the calculated deformation. The system 1 in this example further includes a reporting unit 20. The reporting unit 20 is arranged for making processing results, such as the estimated risk knowable to a user. The reporting unit 20 can e.g. be connected to a visualization device 22, such as a monitor or a printer. Alternatively, or additionally, the reporting unit can be connected to a communications network, such as the internet. The reporting unit 20 can be arranged for creating a numerical report, and or an image-containing report.

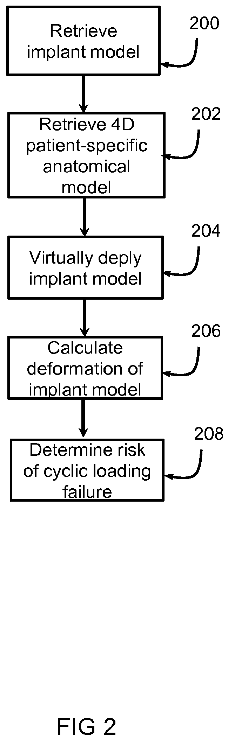

The system 1 can be used as follows in a method for patient-specific predicting of cyclic loading failure of a cardiac implant 2, see Figure 2. In step 200 the implant model 6 is retrieved from the first source 8. In step 202 the 4D patient-specific anatomical model 12 is retrieved from the second source 14. In step 204 the processor 16 virtually deploys the implant model 6 at the deployment site in the 4D patient-specific anatomical model 12. In this example, the implant model 6 is virtually deployed in the 4D patient-specific anatomical model while the 4D anatomical model 12 is stationary. Alternatively, it is possible that the implant model 6 is virtually deployed in the 4D patient-specific anatomical model 12 while the 4D anatomical model 12 transforms consecutively through the plurality of stages, i.e. during beating of the heart.

In step 206 the processor 16 calculates deformation of the implant model 6 deployed at the deployment site when the 4D patient-specific anatomical model 12 transforms consecutively through the plurality of states. Thereto, the processor 16 can calculate e.g. the shape and optionally position, of the implant model 6 deployed in the 4D patient-specific anatomical model 12 for each of the plurality of states. The deformation of the implant model 6 can follow from the shape of the implant model 6 in each of the states.

For determining the deformation of the implant model 6 in each of the states, deformation of the 4D patient-specific anatomical model 12 can be taken as a starting point. Going from one state to the next, the 4D patient-specific anatomical model 12 contains information representative of a displacement for each node of the 4D patient-specific anatomical model 12. The displacement of each node of the 4D patient-specific anatomical model 12 is used for determining the displacement of each node of the implant model 6. This can be performed for each of the states.

In a first example, the 4D patient-specific anatomical model 12 represents a mesh-based representation of a patient-specific cardiac region in a plurality of states corresponding to a plurality of moments in the cardiac cycle before deployment of the implant device. Hence, nodes of the 4D anatomical model 12 transforms through the plurality of states following displacement of the patient-specific cardiac region in the absence of an implant device. Herein, the deformation of the patient-specific anatomical model 12 representing the patient-specific cardiac region is imposed onto the implant model 6 though contact between the anatomical model 12 and the implant model 6. The displacements of each of the nodes of the 4D patient-specific anatomical model 12 are applied to the all or some of the nodes of the implant model 6, depending on mechanical contact between the two models 6, 12. Thus, deformation of the implant model 6 is determined, here for each of the plurality of states, in this example using finite element analysis, FEA. Hence deformation of the patient-specific cardiac region as a result of the presence of the implant device is not taken into account. It has been found that this can provide a good approximation for relatively flexible implant devices.

In a second example, a 4D intermediate patient-specific anatomical model is provided, having associated nodes associated with nodes of the 4D patient-specific anatomical model. The 4D intermediate patient-specific anatomical model represents a mesh-based representation of a patient-specific cardiac region in a plurality of states corresponding to a plurality of moments in the cardiac cycle before deployment. Hence, the associated nodes of the 4D intermediate anatomical model transform through the plurality of states following displacement of the patient-specific cardiac region in the absence of an implant device. The 4D patient-specific anatomical model 12 transforms through the plurality of states through transferring the displacements of associated nodes of the 4D intermediate model to the nodes of the patient-specific anatomical model 12 by means of stiffness elements (e.g. spring-dashpot systems) attached to some or all of the nodes of the mesh of the 4D patient-specific anatomical model 12. Hence, the 4D patient-specific anatomical model 12 can (resiliently) move relative to the associated nodes of the 4D intermediate patient-specific anatomical model. Deformation of the implant model 6 is achieved by contact between 4D patient-specific anatomical model 12 and the implant model 6. Thus, deformation of the implant model 6 is determined, here for each of the plurality of states, in this example using finite element analysis, FEA. Hence deformation of the patient-specific cardiac region as a result of the presence of the implant device is taken into account. It has been found that this can provide a good approximation for relatively stiff implant devices.

In step 208 the processor 16 determines a risk of cyclic loading failure of the cardiac implant on the basis of the calculated displacement (deformation and/or motion) of the implant model 6.

In an example, the processor 16 determines mechanical stress and/or strain within each mesh element of the implant model 6. The processor 16 can e.g. determine mechanical stress and/or strain within each mesh element of the implant model 6 for each of the plurality of states in the course of the cardiac cycle. The mechanical stress and/or strain within the mesh elements of the implant model 6 is representative for cyclic loading fatigue. The mechanical stress and/or strain within the mesh elements of the implant model, and the variation thereof during the entire cycle, is representative for cyclic loading fatigue. The risk of cyclic loading failure can e.g. be determined by means of a rainflow-counting algorithm, as is known in the art.

In an example, the processor 16 determines for each mesh element of the implant model 6 an amplitude of the mechanical stress and/or strain occurring in the course of the cardiac cycle. For each mesh element, a maximum value of the stress and/or strain can be determined among the plurality of states. For each mesh element, a minimum value of the stress and/or strain can be determined among the plurality of states. The amplitude for each mesh element can be determined as a difference between the respective maximum and minimum values. The determined amplitudes are representative of the risk of cyclic loading failure. The risk of cyclic loading failure can e.g. be determined by means of an S-N curve, as is known in the art.

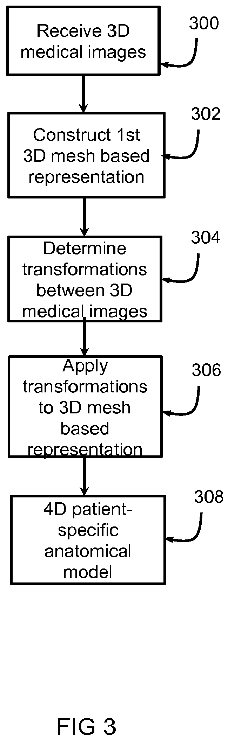

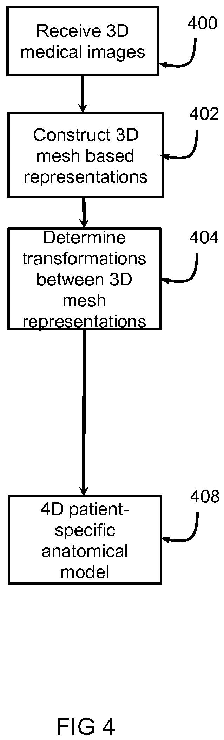

In step 202 the 4D patient-specific anatomical model 12 was retrieved from the second source 14. The 4D patient-specific anatomical model can for example be constructed as follows. Construction of the 4D patient-specific anatomical model can be performed by a construction unit 18 using a method as shown in Figure 3 or 4. The construction unit 18 can be integrated in the processor 16, can be a dedicated unit of the system 1, or can be a system independent of the system 1. In step 300, 400 the construction unit 18 receives a plurality of, at least quasi, 3D medical images. Each 3D medical image represents the patient-specific cardiac region in one of the consecutive states corresponding to the plurality of moments in the cardiac cycle. Together, the plurality of 3D medical images represents the patient-specific cardiac region in all of the plurality of states. The construction unit constructs the 4D patient-specific anatomical model on the basis of the plurality of 3D medical images.

In a first example, the construction unit 18, in step 302, takes a first one of the 3D medical images and constructs a first 3D mesh based representation of the patient-specific cardiac region on the basis of that medical image. Further, in step 304 the construction unit determines for each of the medical images a transformation from one medical image to the next, such as a BSpline transformation. Hence, a plurality of transformations is obtained, each suitable for transforming one of the medical images into the next medical image (a first transformation for transforming the first medical image into the second medical image, a second transformation for transforming the second medical image into the third medical image, and so on). In step 306 the first transformation is applied to the first 3D mesh based representation to obtain a second 3D mesh based representation, the second transformation is applied to the second 3D mesh based representation to obtain a third 3D mesh based representation, and so on. Hence, a plurality of 3D mesh based representations is obtained, one associated with each state in the cardiac cycle. In step 308 the plurality of 3D mesh based representations together form the 4D patient-specific anatomical model.

In a second example, the construction unit 18, in step 402, takes each of the 3D medical images and constructs a 3D mesh based representation for each of the 3D medical images. Further, in step 404 the construction unit 18 determines for each of the 3D mesh based representations a transformation from one representation to the next. Hence, a plurality of transformations is obtained, each suitable for transforming one of the 3D mesh based representations into the next 3D mesh based representation (a first transformation for transforming the first 3D mesh based representation into the second 3D mesh based representation, a second transformation for transforming the second 3D mesh based representation into the third 3D mesh based representation, and so on). Hence, a plurality of 3D mesh based representations is obtained, one associated with each state in the cardiac cycle. In step 408 the plurality of 3D mesh based representations and their transformations together form the 4D patient-specific anatomical model.

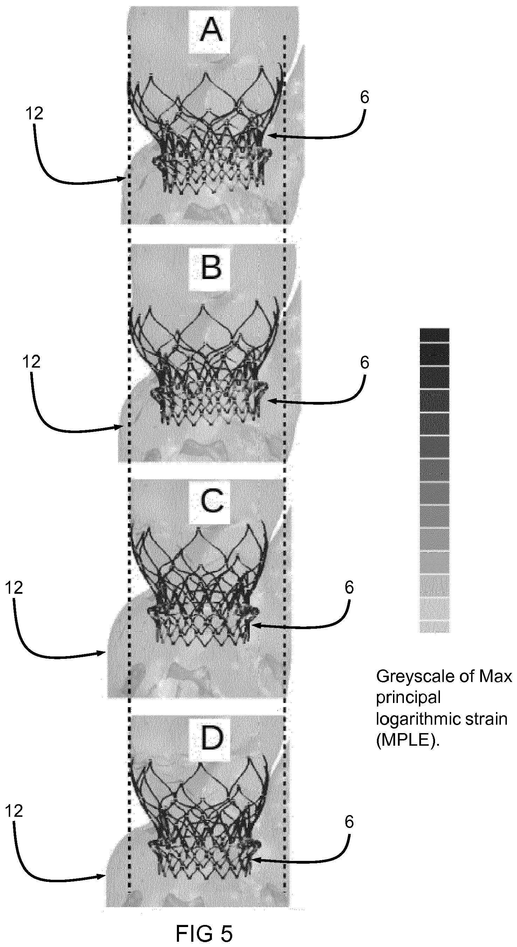

Figures 5A-5D show exemplary examples of an implant model 6, here representing a replacement cardiac valve, deployed in a 4D patient-specific anatomical model 12 at four consecutive states during the cardiac cycle. The deformation of the implant model 6 can easily be observed. Strain in the implant device 6 is indicated in Figures 5A-5D in false colors (the greyscale represents the max principal logarithmic strain (MPLE) of the implant model 6, white indicates MPLE ≤ 0.01%, black indicates MPLE ≥ 0.1%).

It will be appreciated that it is also possible that a plurality of different implant models 6 is provided. Each implant model 6 can represent geometrical and/or material properties of a corresponding real-life implant. The implant models 6 may e.g. differ in size, brand, construction, material or the like. Each of the implant models can then be placed into the patient specific anatomical model 12. The cyclic loading failure risk is then determined for each of the implant models 6. From this analysis it can be determined which one of the plurality of implant models has associated therewith the lowest risk of cyclic loading failure. A cardiac valve implant corresponding to the implant model 6 having the lowest associated risk of cyclic loading failure can then be selected for a real-life percutaneous implantation procedure. It will be appreciated that it is also possible that each of the implant models 6 is placed into the patient-specific anatomical model 12 at a plurality of different locations. Thus, for each implant model a position of risk of cyclic loading failure can de determined. The lowest risk of cyclic loading failure per implant model 6 can then be compared to select the cardiac valve implant for real-life percutaneous implantation.

It will be appreciated that it is also possible that each of the implant models 6 is placed into the patient-specific anatomical model 12 at a plurality of different locations and analyzed for each of the views. Thus for each implant model a lowest risk of cyclic loading failure can be determined among the different locations. Also for each implant model a highest risk of cyclic loading failure can be determined among the different locations. The lowest and highest risk of cyclic loading failure per implant model 6 can then be compared to select the cardiac valve implant for real-life percutaneous implantation. Alternatively, the highest risk of cyclic loading failure can be used to design the bench and calibrate the bench loading conditions for experimental fatigue testing (accelerated wear testing, AWT, of fatigue machine). From patient-specific deformation or stress or strain of the device the condition leading to highest risk of cyclic loading failure can be reproduced with a simplified bench that runs accelerated cycles and allows to test the device fatigue safety within a time period shorter than the in-vivo time period, by performing cycles at higher frequency.

Herein, the invention is described with reference to specific examples of embodiments of the invention. It will, however, be evident that various modifications and changes may be made therein, without departing from the essence of the invention. For the purpose of clarity and a concise description features are described herein as part of the same or separate embodiments, however, alternative embodiments having combinations of all or some of the features described in these separate embodiments are also envisaged.

In general is provided a method for patient-specific predicting of cyclic loading failure of an implant device. The method includes providing an implant model representing a three dimensional mesh based representation of the implant device. The method also includes providing a four-dimensional, 4D, patient-specific anatomical model representing a mesh based representation of a patient-specific anatomical region including a deployment site for the implant device in a plurality of states corresponding to a plurality of moments in a movement cycle, such as the cardiac cycle. According to the method, a computer calculates deformation of the implant model deployed at the deployment site when the 4D patient-specific anatomical model transforms consecutively through the plurality of states, and determines a risk of cyclic loading failure of the implant device on the basis of the calculated deformation. This provides the advantage that cyclic loading failure for the implant device can be predicted on the basis of the patient-specific anatomy and before deployment of the implant device. It will be appreciated that if the movement is not strictly cyclic, nevertheless a representative movement cycle can be defined.

It will be appreciated that the method can be used also after implantation of the device, for example by starting from preoperative medical images and introducing measurements taken after implantation.

It will be appreciated that the processor, first receiving unit, second receiving unit, construction unit, and/or reporting unit can be embodied as dedicated electronic circuits, possibly including software code portions. The processor, first receiving unit, second receiving unit, construction unit, and/or reporting unit can also be embodied as software code portions executed on, and e.g. stored in, a memory of, a programmable apparatus such as a computer, tablet or smartphone.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means, e.g. via the internet or cloud.

When a program is embodied in a signal which may be conveyed directly by a cable or other device or means, the carrier may be constituted by such cable or other device or means. Alternatively, the carrier may be an integrated circuit in which the program is embedded, the integrated circuit being adapted for performing, or for use in the performance of, the relevant processes.

However, other modifications, variations, and alternatives are also possible. The specifications, drawings and examples are, accordingly, to be regarded in an illustrative sense rather than in a restrictive sense.

For the purpose of clarity and a concise description features are described herein as part of the same or separate embodiments, however, it will be appreciated that the scope of the invention may include embodiments having combinations of all or some of the features described.

In the claims, any reference sign placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

## Claims

1. A method for patient-specific predicting of cyclic loading failure of a cardiac implant, including:
- providing an implant model representing a three dimensional mesh based representation of a cardiac implant;
- providing a four-dimensional, 4D, patient-specific anatomical model representing a mesh based representation of a patient-specific cardiac region including a deployment site for the cardiac implant in a plurality of states corresponding to a plurality of moments in the cardiac cycle;
- having a computer calculate, while the 4D patient-specific anatomical model transforms consecutively through the plurality of states, deformation of the implant model deployed at the deployment site; and
- have the computer determine a risk of cyclic loading failure of the cardiac implant on the basis of the calculated implant deformation.

2. The method of claim 1, including having the computer determine a risk of cyclic loading failure of the cardiac implant on the basis of the calculated implant deformation and data representative of deformation history.

3. The method of claim 1, wherein the 4D patient-specific anatomical model represents a mesh-based representation of a patient-specific cardiac region in a plurality of states corresponding to a plurality of moments in the cardiac cycle before deployment.

4. The method of claim 1 or 2, including providing a 4D patient-specific intermediate model, having associated nodes associated with nodes of the 4D patient-specific anatomical model, wherein the 4D patient-specific intermediate model represents a mesh-based representation of a patient-specific cardiac region in a plurality of states corresponding to a plurality of moments in the cardiac cycle before deployment, wherein the patient-specific anatomical model transforms through the plurality of states through transferring the displacements of associated nodes of the 4D intermediate model via stiffness and/or dashpot elements to the nodes of the 4D patient-specific anatomical model.

5. The method of claim 4, wherein the 4D patient-specific anatomical model has mechanical properties, including stiffness and/or viscosity, the overall mechanical behavior of the 4D patient-specific anatomical model depending on the combination of the mechanical properties of the 4D anatomical model and mechanical properties of the stiffness and/or dashpot elements connecting associated nodes to nodes of the 4D anatomical model.

6. The method of any one of claims 1-5, including determining mechanical stress and/or strain within each mesh element of the implant model.

7. The method of claim 6, including determining mechanical stress and or strain within each mesh element of the implant model for each of the plurality of states in the course of the cardiac cycle.

8. The method of claim 7, including determining for each mesh element of the implant model an amplitude of the mechanical stress and/or strain occurring in the course of the cardiac cycle, and determining the risk of cyclic loading failure on the basis of the determined amplitudes.

9. The method of any one of claims 1-8, wherein the cardiac implant is a valve implant, stent, or the like.

10. The method of any one of claims 1-9, wherein the cyclic loading failure includes one or more of high cycle fatigue fracture, implant migration, valve failure.

11. The method of any one of claims 1-10, including providing the 4D patient-specific anatomical model, or its associated nodes, on the basis of one or more of :
- segmentation of a 4D preoperative image;
- landmarks in 4D preoperative images;
- a 3D or 4D preoperative image in combination with patient-specific measurements of blood volume, flow and/or pressure taken before and/or after deployment of the implant;
- a 3D or 4D preoperative image in combination with non-patient specific knowledge of patho-physiology of the heart, such as e.g. expected motion; or
- a 3D or 4D preoperative image in combination with non-patient specific knowledge of heart remodeling at chronic stage.

12. The method of any one of claims 1-11, including receiving a plurality of, at least quasi, 3D medical images representing the patient-specific cardiac region in the plurality of states corresponding to the plurality of moments in the cardiac cycle, and constructing the 4D patient-specific anatomical model on the basis thereof.

13. The method of claim 12, including:
- constructing a 3D mesh based representation of the patient-specific cardiac region on the basis of one of the medical images;
- determining a transformation from one medical image to the next; and
- applying the transformation to the constructed 3D mesh based representation, providing the 4D patient-specific anatomical model.

14. The method of claim 9, including:
- for each of the medical images constructing a 3D mesh based representation of the patient-specific cardiac region;
- determining a transformation from one 3D mesh based representation to the next; and
- determining the 4D patient-specific anatomical model on the basis of the plurality of 3D mesh based representations.

15. The method of any one of claims 1-14, including deploying an implant model at a plurality of different positions in the 4D patient-specific anatomical model, determining a risk of cyclic loading failure for each of the positions, and selecting the position associated with the lowest risk for real-life implantation.

16. The method of any one of claims 1-15, including providing a plurality of different implant models, each implant model representing geometrical and/or material properties of a corresponding real-life implant device, determining a risk of cyclic loading failure of the cardiac implant for each implant model, and selecting the implant device associated with the implant model for which the lowest risk was calculated, for real-life implantation.

17. The method of any one of claims 1-16, including using a highest risk of cyclic loading failure to design the bench and calibrate the bench loading conditions for experimental fatigue testing.

18. A system for patient-specific predicting of cyclic loading failure of a cardiac implant, including a processor arranged for:
- receiving an implant model representing a three dimensional mesh based representation of a cardiac implant;
- receiving a four-dimensional, 4D, patient-specific anatomical model representing a mesh based representation of a patient-specific cardiac region including a deployment site for the cardiac implant in a plurality of states corresponding to a plurality of moments in the cardiac cycle;
- calculating deformation of the implant model deployed at the deployment site when, or before, the 4D patient-specific anatomical model transforms consecutively through the plurality of states; and
- determining a risk of cyclic loading failure of the cardiac implant on the basis of the calculated implant deformation.

19. A computer program product including computer implementable instructions which when implemented by a programmable computer cause the computer to:
- retrieve an implant model representing a three dimensional mesh based representation of a cardiac implant;
- retrieve a four-dimensional, 4D, patient-specific anatomical model representing a mesh based representation of a patient-specific cardiac region including a deployment site for the cardiac implant in a plurality of states corresponding to a plurality of moments in the cardiac cycle;
- calculate deformation of the implant model deployed at the deployment site when the 4D patient-specific anatomical model transforms consecutively through the plurality of states; and
- determine a risk of cyclic loading failure of the cardiac implant on the basis of the calculated implant deformation.
